(19) Europäisches Patentamt
European Patent Office
Office européen des brevets

(11) EP 4 446 302 A1

(12) EUROPEAN PATENT APPLICATION

(43) Date of publication:
**16.10.2024 Bulletin 2024/42**

(21) Application number: **23167561.2**

(22) Date of filing: **12.04.2023**

(51) International Patent Classification (IPC):
***C07C 1/24*** *(2006.01)* ***C07C 29/151*** *(2006.01)*
***C07C 45/34*** *(2006.01)* ***C07C 45/89*** *(2006.01)*
***C07C 51/15*** *(2006.01)* ***C07C 51/235*** *(2006.01)*
***C07C 51/54*** *(2006.01)* ***C07C 67/05*** *(2006.01)*
***C25B 1/00*** *(2021.01)* ***C07C 51/56*** *(2006.01)*

(52) Cooperative Patent Classification (CPC):
(C-Sets available)
**C07C 29/1518; C07C 1/20; C07C 45/34; C07C 45/89; C07C 51/12; C07C 51/235; C07C 51/54; C07C 51/56; C07C 67/00; C07C 67/05; C07C 67/08; C25B 1/00;**
C07B 2200/05 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **BASF SE**
**67056 Ludwigshafen am Rhein (DE)**

(72) Inventors:
- **WEISS, Thomas**
  **67056 Ludwigshafen (DE)**
- **WITTELER, Helmut**
  **67056 Ludwigshafen (DE)**
- **HUEFFER, Stephan**
  **67063 Ludwigshafen (DE)**
- **TROPSCH, Juergen**
  **67056 Ludwigshafen (DE)**
- **KOENIG, Christian**
  **67056 Ludwigshafen (DE)**
- **KRUEGER, Marco**
  **67056 Ludwigshafen (DE)**
- **WIDMAIER, Ralf**
  **67056 Ludwigshafen (DE)**

(74) Representative: **Schuck, Alexander**
**Patentanwälte**
**Isenbruck Bösl Hörschler PartG mbB**
**Eastsite One**
**Seckenheimer Landstraße 4**
**68163 Mannheim (DE)**

# (54) SYNTHETIC VINYL ACETATE HAVING LOW DEUTERIUM CONTENT FROM NON-FOSSIL RESOURCES

(57) A Process for making vinyl acetate, comprising the steps:
(a) providing hydrogen with a deuterium content below 90 ppm, based on the total hydrogen content, by water electrolysis using electrical power that is generated at least in part from non-fossil, renewable resources;
(b) providing carbon dioxide;
(c) reacting hydrogen and carbon dioxide in the presence of a catalyst to form methanol with a deuterium content below 90 ppm, based on the total hydrogen content;
(d) reacting methanol from step (c) to form ethylene; and
(e) reacting methanol from step (c) with carbon monoxide to form acetic acid; and/or
(f1) reacting part of the ethylene from step (d) with oxygen and water to give acetaldehyde;
(f2) reacting acetaldehyde from step (f1) with oxygen to give acetic acid;
(g) reacting acetic acid from step (e) and/or step (f1) with ethylene from step (d) to give vinyl acetate.

EP 4 446 302 A1

(52) Cooperative Patent Classification (CPC): (Cont.)

C-Sets
**C07C 1/20, C07C 11/04;**
**C07C 29/1518, C07C 31/04;**
**C07C 45/34, C07C 47/06;**
**C07C 45/89, C07C 49/90;**
**C07C 51/12, C07C 53/08;**
**C07C 51/235, C07C 53/08;**
**C07C 51/54, C07C 53/12;**
**C07C 51/56, C07C 53/12;**
**C07C 67/00, C07C 69/16;**
**C07C 67/05, C07C 69/15;**
**C07C 67/08, C07C 69/14**

## Description

The present invention relates to a process for making methanol having a deuterium content below 90 ppm, based on the total hydrogen content, the methanol obtained thereby as well as to its use.

The present invention further relates to processes for making vinyl acetate, based on methanol having a deuterium content below 90 ppm, comprising the step of reacting acetic acid with ethylene to give vinyl acetate.

The present invention further relates to processes for making vinyl acetate, based on methanol having a deuterium content below 90 ppm, comprising the steps of reacting acetic anhydride with acetaldehyde to form ethyliden diacetate and reacting ethyliden diacetate to give vinyl acetate by thermal elimination of acetic acid.

The present invention further relates to vinyl acetate having a deuterium content below 90 ppm, based on the total hydrogen content.

In the chemical industry methanol serves as a raw material in the production of olefines, formaldehyde, acetaldehyde, acetic acid, methyl acetate, acetic anhydride and vinyl acetate. The conventional production method involves a catalytic process using fossil feedstock such as natural gas or coal.

Synthesis gas (syngas) for the production of methanol can be produced from many sources, including natural gas, coal, biomass, or virtually any hydrocarbon feedstock, by reaction with steam (steam reforming), carbon dioxide (dry reforming) or oxygen (partial oxidation).

Syngas is produced from solid feedstocks via coal gasification. Coal is reacted thereby in a mixture of partial oxidation with air or pure oxygen and gasification with water vapor to give a mixture of carbon monoxide and hydrogen. Via the Boudouard equilibrium carbon monoxide is in equilibrium with carbon and carbon dioxide.

$$2\ C + O_2 \rightarrow 2\ CO,$$

$$C + H_2O \rightarrow CO + H_2,$$

$$C + CO_2 \rightleftharpoons 2\ CO,$$

Furthermore, the water gas shift reaction must be taken into account.

$$CO + H_2O \rightleftharpoons CO_2 + H_2,$$

The exothermic reaction with oxygen provides the necessary energy to achieve the high reaction temperatures for the endothermic gasification reaction of carbon with water vapor.

In pricinple, beside coal, other solid feedstocks (wood, straw) can be used instead.

The most important gaseous educt for producing syngas is natural gas, which is reacted with water vapor via steam reforming. Natural gas provides the highest hydrogen to carbon monoxide ratio.

$$CH_4 + H_2O \rightarrow CO + 3\ H_2,$$

Also liquid educts, such as light naphtha cuts, can be reacted, after sulfur removal, with water vapor via steam reforming.

To produce methanol, the ratio of carbon monoxide to hydrogen in the synthesis gas is adjusted to meet the reaction equation

$$CO + 2\ H_2 \rightarrow CH_3OH,$$

Synthesis gas is mainly produced via steam reforming or partial oxidation of natural gas or via coal gasification. While natural gas is used for the methanol production in North America and in Europe, syngas production is based mainly on coal in China and South Africa. Depending on the carbon monoxide to hydrogen ratio, the product gases are named water gas ($CO + H_2$), synthesis gas ($CO + 2\ H_2$) or spaltgas ($CO + 3\ H_2$). Spaltgas can be hydrogen depleted or carbon monoxide enriched, for example via the water gas shift reaction by adding carbon dioxide and removing water, and water gas can be hydrogen enriched or carbon monoxide depleted in order to obtain synthesis gas.

The synthesis of methanol from $CO_2$ is less exothermic than that starting from synthesis gas, and it also involves

as secondary reaction the reverse water-gas-shift (RWGS). To facilitate methanol synthesis, the CO in syngas is converted to $CO_2$ through the water-gas shift (WGS) reaction

$$CO_2 + 3\,H_2 \rightarrow CH_3OH + H_2O \qquad \Delta H298K = -49.5\text{ kJ mol-1}$$
$$CO_2 + H_2 \rightarrow CO + H_2O \qquad \Delta H298K = 41.2\text{ kJ mol-1}$$

The water-gas equilibrium mentioned above provides the basis to produce $CO_2$-neutral methanol if the $CO_2$ comes from appropriate direct or indirect biogenic sources. According to the reverse water-gas-shift (RWGS) reaction, there is the opportunity of including biogenic $CO_2$ directly to an adapted syngas - methanol -process. Syngas is then converted to methanol e.g. in the ranges of temperature of 250-300°C and pressure of 5 - 10 MPa, using $CuO/ZnO/Al_2O_3$ catalyst.

In that sense $CO_2$ form different biogenic carbon sources could be included into the syngas to form methanol. The biogenic source of $CO_2$ could be from fermentation processes of biomaterial, combustion processes of biomass or waste of biobased materials or form extractive processes of atmospheric $CO_2$, for example by extractive regenerative process steps such as aminic $CO_2$ scrubbing.

Of course, mixtures of $CO_2$ from biogenic and fossil carbon source could be mixed to be used to produce methanol, too.

The natural isotopic abundance of $^{12}C$ is about 98.9%, the natural isotopic abundance of $^{13}C$ is about 1.1 %. The $^{13}C/^{12}C$ isotopic ratio of chemical compounds is given relative to an international standard, the Vienna-Pee-Dee-Belemnite-Standard (V-PDB). The $^{13}C/^{12}C$ isotopic ratio is given as $\delta^{13}C$ value in the unit ‰. The standard per definition has a $\delta^{13}C$ value of 0 %o. Substances having a higher $^{13}C$ content than the standard have positive, substances having a lower $^{13}C$ content than the standard have negative ‰ values.

Fossil based methanol from fossil based synthesis gas has in general $\delta^{13}C$ values ranging from -50 ‰ to - 25 ‰, depending on the fossil feedstock. Methanol based on carbon dioxide captured from ambient air has in general $\delta^{13}C$ values ranging from -10 ‰ to - 2.5 ‰, corresponding the $\delta^{13}C$ values of carbon dioxide captured from ambient air.

In preferred embodiments of the inventive process, the carbon dioxide provided in step (b) has a $^{13}C$-content corresponding to a $\delta^{13}C$ value of > -20 %o. In particular, the carbon dioxide provided in step (b) has a $^{13}C$-content corresponding to a $\delta^{13}C$ value of from -10 to -2.5 ‰.

The invention also relates to methanol with a deuterium content below 90 ppm, based on the total hydrogen content. Preferably, the deuterium content is from 30 to 75 ppm, based on the total hydrogen content.

The methanol with a deuterium content below 90 ppm, preferably from 30 to 75 ppm, based on the total hydrogen content, can be used to prepare ethylene. In general, the obtained ethylene also has a low deuterium content of below 90 ppm, preferably from 30 to 75 ppm. If carbon dioxide is captured from ambient air, the $^{13}C$-content of the obtained ethylene also corresponds to a $\delta^{13}C$ value of in general > -20 ‰, more specifically to a $\delta^{13}C$ value of from -10 to -2.5 ‰.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Polyvinyl acetate (abbreviations PVAC, PVA) is a thermoplastic. Polyvinyl acetate is an amorphous, odourless and tasteless plastic with high light and weather resistance. It is combustible, but not easily flammable. The glass transition temperature of the homopolymer varies between 18 and 45 °C depending on the degree of polymerization. The electrical, mechanical and thermal properties are also largely dependent on the degree of polymerization. The minimum film forming temperature of homopolymer dispersions is about 15 to 18 °C. Polyvinyl acetate is processed in the form of solutions in organic solvents or as a dispersion.

PVA is used as a binder in paints and varnishes. The plastic is also used as an adhesive, for example as white glue (wood glue), wallpaper paste or parquet adhesive. The universal adhesive UHU, which is well known in Germany,

is a forty percent solution of polyvinyl acetate in methyl acetate and acetone. Simple craft glue also often contains mainly polyvinyl acetate and is then called vinyl glue. Other applications include paper manufacturing and coating, textile impregnation, carpet backing or modification of plaster and concrete. In addition, PVA is often a component of chewing gum masses and is used for coating cheese or sausage.

Vinyl acetate is able to form copolymers with a variety of monomers such as ethylene, maleic anhydride, maleic esters, vinyl ethers and allyl ethers. From that copoymers especially the ethylene vinyl acetate copolymers (EVAC) are prepared in huge amounts to produce thermoplastic elastormers and thermoplastic materials. Depending on the ethylene : vinyl acetate ratio a broad variety off application can be addressed. Vinyl acetate contents of up to 7 % are almost exclusively used to improve the properties (especially to increase the elongation at break) of films. About half of EVAC production is made with a vinyl acetate content of less than 7 %. EVAC with a vinyl acetate content of 7 to 18 % is also often used as an exclusive material for special applications. Examples include cold-resistant pull-out spouts for canisters, films for agriculture and horticulture, shrink-wrap films (office supplies, solar panels), shower curtains, floor coverings, roofing membranes and electrical cables. The class of EVAC above with up to 28 % vinyl acetate is mainly used as a hot melt adhesive, which in turn is used for fibre bonding of very high quality tufted carpets and needle punched nonwovens, for perfect binding in book production and for manual use with hot melt glue guns. With vinyl acetate contents of more than 30% up to 90%, a rubber-like thermoplastic elastomer is produced. It is mainly used for shoe soles or as a polymer blend together with other elastomers.

Furthermore the vinyl acetate monomer (VAM) can be used to prepare watersoluble graft-polymers on polyether substrates. These graft-copolymers are used in laundry detergent compositions for antigraying, e.g. Sokalan HP22 (DE3711298; BASF SE).

Vinyl acetate is produced form precursor base stocks such as ethylene and acetic acid. These base stocks typically derive from fossil carbon base stocks, such as oil or natural gas. Since the carbon source leads to an unwanted $CO_2$ balance there is need for production of vinyl acetate monomer (VAM) and polyvinyl acetate and copolymers in a way that does not use fossil carbon sources. Vinyl acetate can form polymers such a polyvinyl acetate which is able to be hydrolyzed to biodegradable polyvinyl alcohol and biodegradable acetic acid. The production of vinyl acetate monomer VAM from non-fossil, renewable hydrogen and carbon sources has not been described before.

It is an object of the present invention to provide an environmentally friendly process for producing methanol. It is a further object of the present invention to provide a methanol having a low deuterium content. The favorable kinetic isotope effect caused by the low deuterium content of the methanol may be cumulative, since it is also present in subsequent production steps further downstream in the value chain.

It is a further object of the present invention to provide an environmentally friendly process for producing vinyl acetate.

The object is solved by a process for making methanol having a deuterium content below 90 ppm, based on the total hydrogen content, comprising the steps:

(a) providing hydrogen with a deuterium content below 90 ppm by water electrolysis using electrical power that is generated at least in part from non-fossil, renewable resources;
(b) providing carbon dioxide;
(c) reacting hydrogen and carbon dioxide in the presence of a catalyst to form methanol.

The object is further solved by a process for making vinyl acetate, comprising the steps:

(a) providing hydrogen with a deuterium content below 90 ppm, based on the total hydrogen content, by water electrolysis using electrical power that is generated at least in part from non-fossil, renewable resources;
(b) providing carbon dioxide;
(c) reacting hydrogen and carbon dioxide in the presence of a catalyst to form methanol with a deuterium content below 90 ppm, based on the total hydrogen content;
(d) reacting methanol from step (c) to form ethylene; and
(e) reacting methanol from step (c) with carbon monoxide to form acetic acid; and/or
(f1) reacting part of the ethylene from step (d) with oxygen and water to give acetaldehyde;
(f2) reacting acetaldehyde from step (f1) with oxygen to give acetic acid;
(g) reacting acetic acid from step (e) and/or step (f1) with ethylene from step (d) to give vinyl acetate.

The object is further solved by a process for making vinyl acetate, comprising the steps:

(a) providing hydrogen with a deuterium content below 90 ppm, based on the total hydrogen content, by water electrolysis using electrical power that is generated at least in part from non-fossil, renewable resources;
(b) providing carbon dioxide;

(c) reacting hydrogen and carbon dioxide in the presence of a catalyst to form methanol with a deuterium content below 90 ppm, based on the total hydrogen content;
(d) reacting methanol from step (c) to form ethylene; and
(e) reacting ethylene from step (d) with oxygen and water to form acetaldehyde;
(f1) reacting part of the acetaldehyde from step (e) with oxygen to form acetic acid; and/or
(f2) reacting methanol from step (c) with carbon monoxide to form acetic acid; and
(g1) reacting acetic acid from step (f1) and/or step (f2) with methanol from step (c) to form methyl acetate;
(g2) reacting methyl acetate from step (g1) with carbon monoxide to form acetic anhydride; and/or
(h1) producing ketene from acetic acid from step (f1) and/or step (f2);
(h2) reacting ketene from step (h1) with acetic acid from step (f1) and/or step (f2) to give acetic acid anhydride;
(i) reacting acetic anhydride form step (g2) and/or step (h2) with acetaldehyde from step (e) to form ethyliden diacetate;
(k) reacting ethyliden diacetate to give vinyl acetate by thermal elimination of acetic acid.

Fossil based methanol from synthesis gas has in general $\delta^{13}C$ values ranging from -50 ‰ to - 25 ‰, depending on the fossil feedstock. Methanol based on carbon dioxide captured from ambient air has in general $\delta^{13}C$ values ranging from -10 ‰ to - 2.5 %o, corresponding to the $\delta^{13}C$ values of carbon dioxide captured from ambient air.

In preferred embodiments of the inventive process, the carbon dioxide provided in step (b) has a $^{13}C$-content corresponding to a $\delta^{13}C$ value of > -20 %o. In particular, the carbon dioxide provided in step (b) has a $^{13}C$-content corresponding to a $\delta^{13}C$ value of from -10 to -2.5 %o.

So if carbon dioxide is captured from ambient air, the $^{13}C$-content of the methanol corresponds to a $\delta^{13}C$ value of in general > -20 ‰, more specifically to a $\delta^{13}C$ value of from -10 to -2.5 ‰. The invention also relates to methanol with a deuterium content below 90 ppm, based on the total hydrogen content. Preferably, the deuterium content is from 30 to 75 ppm, based on the total hydrogen content.

The deuterium content of hydrogen and chemical compounds containing hydrogen is given herein in atom-ppm based on the total hydrogen content (total atoms of protium $^1H$ and deuterium $^2H$).

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general below 90 ppm, preferably from 30 to 75 ppm. The deuterium atom content in electrolytically produced hydrogen may be as low as 15 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 $mA/cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity ($0.1 \pm 0.02$ S $cm^{-1}$), low thickness (20-300 $\mu$m), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind

and solar power, where sudden spikes in energy output would otherwise result in uncaptured energy. The polymer electrolyte allows the PEM water electrolyzer to operate with a very thin membrane (ca. 100-200 $\mu$m) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nafion®, a DuPont product. While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor $\beta$

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number $\lambda$ of between 4 and 9 at the given water mass flow in the anode. The stoichiometric number $\lambda$ is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), $\rho$ is the density of water (g $mL^{-1}$) and $M_{H2O}$ (g $mol^{-1}$) is the molar weight of water. A stoichiometric number $\lambda$ of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at $\lambda = 4$.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably $\leq$ 50%, preferably $\leq$ 30%, most preferably $\leq$ 20%.

The electrical power from non-fossil resources used in water electrolysis according to the invention can be generated by nuclear energy. Nuclear energy is considered renewable by the European Commission, as long as certain preconditions (i. a. safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a pen-stock to drive a turbine.

Wave power, which captures the energy of ocean surface waves, and tidal power, converting the energy of tides, are two forms of hydropower with future potential.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

In step (b) of the inventive process, carbon dioxide is provided. In preferred embodiments, the carbon dioxide that is provided in step (b) is captured from industrial flue gases or from ambient air. All available capture technologies may be used.

Capturing $CO_2$ is most cost-effective at point sources, such as large carbon-based energy facilities, industries with major $CO_2$ emissions (e.g. cement production, steelmaking), natural gas processing, synthetic fuel plants and fossil fuel-based hydrogen production plants. Extracting $CO_2$ from air is possible, although the lower concentration of $CO_2$ in air compared to combustion sources complicates the engineering and makes the process therefore more expensive.

In some preferred embodiments, the carbon dioxide that is provided in step (b) is captured from industrial flue gases.

In post combustion capture, the $CO_2$ is removed after combustion of the fossil fuel-this is the scheme that would apply to fossil-fuel power plants. $CO_2$ is captured from flue gases at power stations or other point sources. Absorption, or carbon scrubbing with amines is the dominant capture technology. It is the only carbon capture technology so far that has been used industrially.

$CO_2$ adsorbs to a MOF (Metal-organic framework) through physisorption or chemisorption based on the porosity and selectivity of the MOF leaving behind a $CO_2$ poor gas stream. The $CO_2$ is then stripped off the MOF using temperature swing adsorption (TSA) or pressure swing adsorption (PSA) so the MOF can be reused.

In some other preferred embodiments, the carbon dioxide that is provided in step (b) is captured from ambient air.

Direct air capture (DAC) is a process of capturing carbon dioxide ($CO_2$) directly from the ambient air and generating a concentrated stream of $CO_2$ for sequestration or utilization or production of carbon-neutral fuel. Carbon dioxide removal is achieved when ambient air makes contact with chemical media, typically an aqueous alkaline solvent or sorbents. These chemical media are subsequently stripped of $CO_2$ through the application of energy (namely heat), resulting in a $CO_2$ stream that can undergo dehydration and compression, while simultaneously regenerating the chemical

media for reuse.

Dilute $CO_2$ can be efficiently separated using an anionic exchange polymer resin called Marathon MSA, which absorbs air $CO_2$ when dry, and releases it when exposed to moisture. A large part of the energy for the process is supplied by the latent heat of phase change of water. Other substances which can be used are metal-organic frameworks (or MOFs). Membrane separation of $CO_2$ rely on semi-permeable membranes.

In step (c), hydrogen and carbon dioxide are reacted in the presence of a catalyst to form methanol.

An overview of suitable catalyst systems is given by Kristian Stangeland, Hailong Li & Zhixin Yu, Energy, Ecology and Environment volume 5, pages 272-285 (2020). Multi-component catalyst systems are required for this process. The interaction between components is essential for high activity and selectivity of $CO_2$-to-methanol catalysts. This has been demonstrated by numerous catalyst systems comprised of various metals (i.e., Cu, Pd, Ni) and metal oxides (i.e., ZnO, $ZrO_2$, $In_2O_3$). These complex systems can contain a mixture of metallic, alloy, and metal oxide phases. The most promising catalyst systems for large-scale industrial processes are currently Cu-based and In-based catalysts due to their superior catalytic performance.

A process for the $CO_2$-to-methanol synthesis can be carried out, for example, by the method known from DE-A-42 20 865, which produces methanol under the influence of silent electrical discharges.

Alternatively, methanol synthesis can also be carried out in a thermal reactor under pressure and elevated temperature and in the presence of a copper-based catalyst (DE 43 32 789 A1; DE 19739773 A1).

Typical catalysts are described, for example, in the publication by N.Kanoun et al. "Catalytic properties of Cu based catalysts containing Zr and/or V for methanol synthesis from a carbon dioxide and hydrogen mixture" in CATALYSIS LETTERS 15,(1992) 231-235. Potential catalysts like CuO/ZnO and Cu-ZnO-$Al_2O_3$ are also described by R. M. Navarro et al. "Methanol Synthesis from CO2: A Review of the Latest Developments in Heterogeneous Catalysis" Materials (2019), 12, 3902 and in "Catalytic carbon dioxide hydrogenation to methanol: A review of recent studies" in chemical engineering research and design 92 (2014) 2557-2567.

Recently a high selective catalysts $In_2O_3/ZrO_2$ has been described for industrial relevant conditions. A typical range of industrially relevant conditions for the hydrogenation of $CO_2$ to methanol are T=200-300°C, p=10-50 MPa, and gas hourly space velocity (GHSV) of 16 000-48000 $h^{-1}$ (Angew. Chem. Int. Ed. 2016, 55, 6261 -6265).

Step (c) can be carried out in the presence of a copper-zinc-alumina catalyst. If copper-zinc-alumina catalysts are employed, the preferred temperature is in the range of from 150 to 300°C, preferably 175 to 300°C and the preferred pressure is in the range of from 10 to 150 bar (abs).

In general, ethylene is produced from methanol in a methanol to olefin-process (MTO-process). Since the process involves the cleavage of C-H bonds and C-D bonds, respectively, the related primary isotope effect will be pronounced. In the MTO process, a mixture of ethylene and propylene is produced from methanol on a highly selective silicon alumina phosphate zeolith-catalyst in fluid bed operation. The ratio propylene to ethylene can be adjusted by chosing appropriate process conditions, and may vary from 0.77 in the ethylene production mode and 1.33 in the propylene production mode.

The overall kinetic isotope effect is cumulative, since it will also be present in all subsequent production steps downstream the value chain.

In environmentally friendly processes of the invention to produce vinyl acetate, vinyl acetate is produced from methanol obtained in step (c) by

(d) reacting methanol from step (c) to form ethylene; and
(e) reacting methanol from step (c) with carbon monoxide to form acetic acid; and/or
(f1) reacting part of the ethylene from step (d) with oxygen and water to give acetaldehyde;
(f2) reacting acetaldehyde from step (f1) with oxygen to give acetic acid;
(g) reacting acetic acid from step (e) and/or step (f1) with ethylene from step (d) to give vinyl acetate.

In step (d), ethylene is produced from methanol in a methanol to olefin-process (MTO-process) as described above.

Acetic acid can be produced by carbonylation of methanol in step (e). The process involves iodomethane as an intermediate, and occurs in three steps. A catalyst, metal carbonyl, is needed for the carbonylation (step 2).

1.

$$CH_3OH + HI \rightarrow CH_3I + H_2O$$

2.

$$CH_3I + CO \rightarrow CH_3COI$$

3.

$$CH_3COI + H_2O \rightarrow CH_3COOH + HI$$

Two related processes exist for the carbonylation of methanol: the rhodium-catalyzed Monsanto process, and the iridium-catalyzed Cativa process.

The Monsanto process operates at a pressure of 30-60 atm and a temperature of 150-200°C and gives a selectivity greater than 99%. The catalytically active species is the anion *cis*-$[Rh(CO)_2I_2]^-$. The first organometallic step is the oxidative addition of methyl iodide to *cis*-$[Rh(CO)_2I_2]^-$ to form the hexacoordinate species $[(CH_3)Rh(CO)_2I_3]^-$. This anion rapidly transforms, via the migration of a methyl group to an adjacent carbonyl ligand, affording the penta-coordinate acetyl complex $[(CH_3CO)Rh(CO)I_3]^-$. This five-coordinate complex then reacts with carbon monoxide to form the six-coordinate dicarbonyl complex, which undergoes reductive elimination to release acetyl iodide ($CH_3C(O)I$). The catalytic cycle involves two non-organometallic steps: conversion of methanol to methyl iodide and the hydrolysis of the acetyl iodide to acetic acid and hydrogen iodide.

The Cativa process is a further method for the production of acetic acid by the carbonylation of methanol. The technology is similar to the Monsanto process. The process is based on an iridium-containing catalyst, such as the complex $[Ir(CO)_2I_2]^-$. The catalytic cycle for the Cativa process begins with the reaction of methyl iodide with the square planar active catalyst species to form the octahedral iridium(III) species $[Ir(CO)_2(CH_3)I_3]^-$. This oxidative addition reaction involves the formal insertion of the iridium(I) centre into the carbon-iodine bond of methyl iodide. After ligand exchange of iodide for carbon monoxide, the migratory insertion of carbon monoxide into the iridium-carbon bond results in the formation of a species with a bound acetyl ligand. The active catalyst species is regenerated by the reductive elimination of acetyl iodide. The acetyl iodide is hydrolysed to produce the acetic acid product, in the process generating hydroiodic acid which is in turn used to convert the starting material methanol to the methyl iodide used in the first step.

The Wacker process or the Wacker-Hoechst process refers to the oxidation of ethylene to acetaldehyde in the presence of palladium(II) chloride as the catalyst.

The net reaction can be described as follows:

$$[PdCl_4]^{2-} + C_2H_4 + H_2O \rightarrow CH_3CHO + Pd + 2\ HCl + 2\ Cl^-$$

This conversion is followed by reactions that regenerate the Pd(II) catalyst:

$$Pd + 2\ CuCl_2 + 2\ Cl^- \rightarrow [PdCl_4]^{2-} + 2\ CuCl$$

$$2\ CuCl + 1/2\ O_2 + 2\ HCl \rightarrow 2\ CuCl_2 + H_2O$$

Two routes are commercialized for the production of acetaldehyde: one-stage processes and two-stage processes.

In the one-stage process, ethene and oxygen are passed co-currently in a reaction tower at about 130°C and 400 kPa. The catalyst is an aqueous solution of $PdCl_2$ and $CuCl_2$. The acetaldehyde is purified by extractive distillation followed by fractional distillation. Extractive distillation with water removes the lights ends having lower boiling points than acetaldehyde (chloromethane, chloroethane, and carbon dioxide) at the top, while water and higher-boiling byproducts, such as acetic acid, crotonaldehyde or chlorinated acetaldehydes, are withdrawn together with acetaldehyde at the bottom.

In the two-stage process, reaction and oxidation are carried out separately in tubular reactors. Unlike the one-stage process, air can be used instead of oxygen. Ethylene is passed through the reactor along with catalyst at 105 - 110°C and 900 - 1000 kPa. Catalyst solution containing acetaldehyde is separated by flash distillation. The catalyst is oxidized in the oxidation reactor at 1000 kPa using air as oxidizing medium. Oxidized catalyst solution is separated and sent back to the reactor. Oxygen from air is used up completely and the exhaust air is circulated as inert gas. Acetaldehyde water vapor mixture is preconcentrated to 60 - 90% acetaldehyde by utilizing the heat of reaction and the discharged water is returned to the flash tower to maintain the catalyst concentration. A two-stage distillation of the crude acetaldehyde follows. In the first stage, low-boiling substances, such as chloromethane, chloroethane and carbon dioxide, are separated. In the second stage, water and higher-boiling by-products, such as chlorinated acetaldehydes and acetic acid, are removed and acetaldehyde is obtained in pure form overhead.

In both one- and two-stage processes the acetaldehyde yield is about 95%.

For further details, reference can be made to Marc Eckert, Gerald Fleischmann, Reinhard Jira, Hermann M. Bolt, Klaus Golka, Acetaldehyd, in Ullmann's Encyclopedia of Industrial Chemistry-7th ed, Vol.1, Kap. 4.3, p. 197.

Acetaldehyde can be oxidized with pure oxygen or with air in the presence of a redox catalyst to give acetic acid. The oxidation can be carried out in the presence of cobalt or manganese acetate in acetic acid as solvent at temperatures of 50 - 70°C in bubble columns (Hoechst process).

Most of the vinyl acetate is produced via the vapor-phase reaction of ethylene and acetic acid over a noble-metal catalyst, usually palladium. The reaction is typically carried out at 150 - 250°C, preferably 175 - 200°C and 5-9 bar pressure. The reaction is usually performed in the gas phase in a fixed bed tubular reactor using a supported catalyst. The amount of oxygen in the combined feed is within the range of 5 to 15 mol %. Preferably, the amount of acetic acid in the combined feed is within the range of 10 to 25 mol %. Preferably, the amount of ethylene in the combined feed is within the range of 65 to 80 mol %. Suitable catalysts include those known to the vinyl acetate industry. Preferably, the catalyst is a palladium-gold catalyst. Methods for preparing palladium-gold catalysts are known. For instance, U.S. Pat. No. 6,022,823 teaches how to prepare a palladium-gold catalyst which has high activity and selectivity. Preferably, the palladium-gold catalyst is supported on an inorganic oxide, such as alumina, silica, titania, and the like, and mixtures thereof.

In further environmentally friendly processes of the invention, vinyl acetate is produced from methanol obtained in step (c) by

(d) reacting methanol from step (c) to form ethylene; and
(e) reacting ethylene from step (d) with oxygen and water to form acetaldehyde;
(f1) reacting part of the acetaldehyde from step (e) with oxygen to form acetic acid; and/or
(f2) reacting methanol from step (c) with carbon monoxide to form acetic acid; and
(g1) reacting acetic acid from step (f1) and/or step (f2) with methanol from step (c) to form methyl acetate;
(g2) reacting methyl acetate from step (g1) with carbon monoxide to form acetic anhydride; and/or
(h1) producing ketene from acetic acid from step (f1) and/or step (f2);
(h2) reacting ketene from step (h1) with acetic acid from step (f1) and/or step (f2) to give acetic acid anhydride;
(i) reacting acetic anhydride form step (g2) and/or step (h2) with acetaldehyde from step (e) to form ethyliden diacetate;
(k) reacting ethyliden diacetate to give vinyl acetate by thermal elimination of acetic acid.

This further route to vinyl acetate involves the reaction of acetaldehyde and acetic anhydride, in general in the presence of a ferric chloride catalyst, to give ethyliden diacetate (step (i)):

$$CH_3CHO + (CH_3CO)_2O \rightarrow (CH_3CO_2)_2CHCH_3.$$

The reaction can be carried out in liquid phase at 120 - 140°C. Ethyliden diacetate can be converted to vinyl acetate by thermal elimination of acetic acid (step (k)):

$$(CH_3CO_2)_2CHCH_3 \rightarrow CH_3CO_2CH{=}CH_2 + CH_3CO_2H.$$

For further details, reference is made to G. Roscher, "Vinyl Esters", in Ullmann's Encyclopedia of Chemical Technology, 2007 John Wiley & Sons, New York. doi:10.1002/14356007.a27_419.

Acetic acid can be reacted with methanol to give methyl acetate in step (g1).

For further details, reference can be made to Aslam, M., Torrence, G.P. and Zey, E.G. (2000), Esterification, in Kirk-Othmer Encyclopedia of Chemical Technology Vol.10, p.471; Le Berre, C., Serp, P., Kalck, P. and Torrence, G.P. (2014), Acetic Acid, in Ullmann's Encyclopedia of Industrial Chemistry, 7th ed, Kap.10.2.1, p. 25.

Esters are most commonly prepared by the reaction of a carboxylic acid and an alcohol with the elimination of water. Esters are also formed by a number of other reactions utilizing acid anhydrides, acid chlorides, amides, nitriles, unsaturated hydrocarbons, ethers, aldehydes, ketones, alcohols, and esters (via ester interchange). In making acetate esters, the primary alcohols are esterified most rapidly and completely, ie, methanol gives the highest yield and the most rapid reaction. Most commercially available methyl acetate is a byproduct in the manufacture of acetic acid. Another method is the esterification of methanol and acetic acid with sulfuric acid as catalyst. The product ester is removed as the methanol/methyl acetate azeotrope

Acetic anhydride can be produced in step (g2) by carbonylation of methyl acetate:

$$CH_3CO_2CH_3 + CO \rightarrow (CH_3CO)_2O$$

This process is known as Tennessee Eastman acetic anhydride process and involves the conversion of methyl acetate to methyl iodide and an acetate salt. Carbonylation of the methyl iodide in turn affords acetyl iodide, which reacts with acetate salts or acetic acid to give the product. Rhodium chloride in the presence of lithium iodide is employed as catalyst. Because acetic anhydride is not stable in water, the conversion is conducted under anhydrous conditions.

For further details, reference can be made to Held, H., Rengstl, A. and Mayer, D. (2000), Acetic Anhydride and Mixed Fatty Acid Anhydrides, in Ullmann's Encyclopedia of Industrial Chemistry, 7th ed, Vol.1, Kap.1.3.3, p. 248 ff.

Acetic anhydride can also be prepared in step (h2) by the reaction of ketene with acetic acid, for example at 45 - 55°C and low pressure (0.05 - 0.2 bar). Ketene can be produced in step (h1) by the dehydration of acetic acid at 700 - 750°C.

For further details, reference can be made to Held, H., Rengstl, A. and Mayer, D. (2000), Acetic Anhydride and Mixed Fatty Acid Anhydrides, in Ullmann's Encyclopedia of Industrial Chemistry, 7th ed, Vol.1, Kap.1.3.1, p. 245 ff.

Acetic anhydride can be also obtained directly by liquid-phase oxidation of acetaldehyde in step (f1). The peracetic acid formed from oxygen and acetaldehyde reacts under suitable conditions with a second molecule of acetaldehyde to form acetic anhydride and water. In practice, a 1 : 2 mixture of acetaldehyde and ethyl acetate is oxidized with the addition of 0.05 to 0.1 % cobalt acetate and copper acetate at 40°C; the ratio of Co:Cu is 1:2. The ratio of acetic anhydride to acetic acid obtained is 56:44, whereas on oxidizing in the absence of ethyl acetate this ratio is only 20:80.

For further details, reference can be made to Held, H., Rengstl, A. and Mayer, D. (2000), Acetic Anhydride and Mixed Fatty Acid Anhydrides, in Ullmann's Encyclopedia of Industrial Chemistry, 7th ed, Vol.1, Kap.1.3.2, p. 244 ff.

The invention further concerns vinyl acetate with a deuterium content below 90 ppm, based on the total hydrogen content, obtainable by the processes as described herein.

The vinyl acetate according has preferably a deuterium content of from 30 to 75 ppm, based on the total hydrogen content.

The vinyl acetate according can have a $^{13}C$-content corresponding to a $\delta^{13}C$ value of from -10 to -2.5 ‰, if the carbon dioxide of step (b) is captured from ambient air.

Vinyl acetate can be polymerized to polyvinyl acetate. Polyvinyl acetate is finally hydrolyzed to polyvinyl alcohol, which is biodegradable. Biodegradation of polyvinyl alcohol yields $H_2O$ and $CO_2$, thereby closing the loop for a $CO_2$-neutral life cycle, if $CO_2$ is taken from the atmosphere in step (b).

Poly(vinyl esters) are nontoxic but are degraded slowly in water, see Rinno, H. (2000), Poly(vinyl esters), in Ullmann's Encyclopedia of Industrial Chemistry, 7th ed, Vol. 28, Kap.8, p. 477 ff.

Polyvinyl alcohol is recognised as one of the very few vinyl polymers soluble in water that is susceptible to ultimate biodegradation in the presence of suitably acclimated microorganisms. Polyvinyl alcohol is nontoxic but is expected to biodegrade within 90 d under aquatic conditions, see Dominic Byrne et al., Biodegradability of polyvinyl alcohol based film used for liquid detergent capsules, Tenside Surf. Det. 58 (2021) 2; E. Chiellini et al., Prog. Polym. Sci. 28 (2003), pp. 963-1014.

**Claims**

1. Process for making vinyl acetate, comprising the steps:

   (a) providing hydrogen with a deuterium content below 90 ppm, based on the total hydrogen content, by water electrolysis using electrical power that is generated at least in part from non-fossil, renewable resources;
   (b) providing carbon dioxide;
   (c) reacting hydrogen and carbon dioxide in the presence of a catalyst to form methanol with a deuterium content below 90 ppm, based on the total hydrogen content;
   (d) reacting methanol from step (c) to form ethylene; and
   (e) reacting methanol from step (c) with carbon monoxide to form acetic acid; and/or
   (f1) reacting part of the ethylene from step (d) with oxygen and water to give acetaldehyde;
   (f2) reacting acetaldehyde from step (f1) with oxygen to give acetic acid;
   (g) reacting acetic acid from step (e) and/or step (f1) with ethylene from step (d) to give vinyl acetate.

2. The process of claim 1, wherein the electrical power is generated from wind power, solar energy, biomass, hydropower or geothermal energy.

3. The process according to claim 1 or 2, wherein hydrogen is provided by polymer electrolyte membrane water electrolysis, preferably by proton exchange membrane water electrolysis (PEMWE) or anion exchange membrane water electrolysis (AEMWE).

4. The process according to any one of claims 1 to 3, wherein the carbon dioxide that is provided in step (b) is captured from industrial flue gases or is captured form ambient air.

5. The process according to any one of claims 1 to 4, wherein the hydrogen provided in step (a) has a deuterium

content of from 30 to 75 ppm.

6. The process according to any one of claims 1 to 5, wherein the carbon dioxide provided in step (b) has a $^{13}C$-content corresponding to a $\delta^{13}C$ value of from -10 to -2.5 %o.

7. Vinyl acetate with a deuterium content below 90 ppm, based on the total hydrogen content, obtainable by the process according to any one of claims 1 to 6.

8. Vinyl acetate according to claim 7 with a deuterium content of from 30 to 75 ppm, based on the total hydrogen content.

9. Vinyl acetate according to claim 7 or 8 having a $^{13}C$-content corresponding to a $\delta^{13}C$ value of from -10 to -2.5 %o.

10. Process for making vinyl acetate, comprising the steps:

(a) providing hydrogen with a deuterium content below 90 ppm, based on the total hydrogen content, by water electrolysis using electrical power that is generated at least in part from non-fossil, renewable resources;
(b) providing carbon dioxide;
(c) reacting hydrogen and carbon dioxide in the presence of a catalyst to form methanol with a deuterium content below 90 ppm, based on the total hydrogen content;
(d) reacting methanol from step (c) to form ethylene; and
(e) reacting ethylene from step (d) with oxygen and water to form acetaldehyde;
(f1) reacting part of the acetaldehyde from step (e) with oxygen to form acetic acid; and/or
(f2) reacting methanol from step (c) with carbon monoxide to form acetic acid; and
(g1) reacting acetic acid from step (f1) and/or step (f2) with methanol from step (c) to form methyl acetate;
(g2) reacting methyl acetate from step (g1) with carbon monoxide to form acetic anhydride; and/or
(h1) producing ketene from acetic acid from step (f1) and/or step (f2);
(h2) reacting ketene from step (h1) with acetic acid from step (f1) and/or step (f2) to give acetic acid anhydride;
(i) reacting acetic anhydride form step (g2) and/or step (h2) with acetaldehyde from step (e) to form ethyliden diacetate;
(k) reacting ethyliden diacetate to give vinyl acetate by thermal elimination of acetic acid.

11. The process of claim 10, wherein the electrical power is generated from wind power, solar energy, biomass, hydropower and geothermal energy.

12. The process according to claim 10 or 11, wherein hydrogen is provided by polymer electrolyte membrane water electrolysis, preferably by proton exchange membrane water electrolysis (PEMWE) or anion exchange membrane water electrolysis (AEMWE).

13. The process according to any one of claims 10 to 12, wherein the carbon dioxide that is provided in step (b) is captured from industrial flue gases or is captured form ambient air.

14. The process according to any one of claims 10 to 13, wherein the hydrogen provided in step (a) has a deuterium content of from 30 to 75 ppm.

15. The process according to any one of claims 10 to 14, wherein the carbon dioxide provided in step (b) has a $^{13}C$-content corresponding to a $\delta^{13}C$ value of from -10 to -2.5 %o.

16. Vinyl acetate with a deuterium content below 90 ppm, based on the total hydrogen content, obtainable by the process according to any one of claims 10 to 15.

17. Vinyl acetate according to claim 16 with a deuterium content of from 30 to 75 ppm, based on the total hydrogen content.

18. Vinyl acetate according to claim 16 or 17 having a $^{13}C$-content corresponding to a $\delta^{13}C$ value of from -10 to -2.5 ‰.

Europäisches Patentamt
European Patent Office
Office européen des brevets

# EUROPEAN SEARCH REPORT

Application Number
EP 23 16 7561

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 6 781 014 B1 (VIDALIN KENNETH EBENES [CA] ET AL) 24 August 2004 (2004-08-24)<br>* claim 6; figure 2 * | 7-9, 16-18<br>1-6, 10-15 | INV.<br>C07C1/24<br>C07C29/151<br>C07C45/34<br>C07C45/89<br>C07C51/15<br>C07C51/235<br>C07C51/54<br>C07C67/05<br>C25B1/00<br>C07C51/56 |
| X<br>A | US 2010/168466 A1 (JOHNSTON VICTOR J [US] ET AL) 1 July 2010 (2010-07-01)<br>* [0024] -[0028];<br>claim 1 * | 7-9, 16-18<br>1-6, 10-15 | |
| A | GB 2 464 691 A (WALL CHRISTOPHER DENHAM [GB]) 28 April 2010 (2010-04-28)<br>* claim 4 * | 1-18 | |

TECHNICAL FIELDS SEARCHED (IPC)

C07C

The present search report has been drawn up for all claims

1

| Place of search | Date of completion of the search | Examiner |
|---|---|---|
| The Hague | 21 September 2023 | Hacking, Michiel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 16 7561

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2023

| Patent document cited in search report | | Publication date | Patent family member(s) | | | Publication date |
|---|---|---|---|---|---|---|
| US 6781014 | B1 | 24-08-2004 | NONE | | | |
| US 2010168466 | A1 | 01-07-2010 | AR | 081505 | A1 | 03-10-2012 |
| | | | BR | PI0923838 | A2 | 21-07-2015 |
| | | | CA | 2748552 | A1 | 08-07-2010 |
| | | | CL | 2011001611 | A1 | 30-09-2011 |
| | | | CN | 102272088 | A | 07-12-2011 |
| | | | CN | 102775300 | A | 14-11-2012 |
| | | | EP | 2382180 | A1 | 02-11-2011 |
| | | | JP | 2012514034 | A | 21-06-2012 |
| | | | SG | 172799 | A1 | 29-08-2011 |
| | | | TW | 201031633 | A | 01-09-2010 |
| | | | US | 2010168466 | A1 | 01-07-2010 |
| | | | WO | 2010077342 | A1 | 08-07-2010 |
| GB 2464691 | A | 28-04-2010 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 3711298
- DE 4220865 A
- DE 4332789 A1
- DE 19739773 A1
- US 6022823 A


### Non-patent literature cited in the description


- **S. KUMAR ; V. HIMABINDU.** *Material Science for Energy Technologies,* 2019, vol. 2, 4442-4454
- **H. A. MILLER et al.** *Sustainable Energy Fuels,* 2020, vol. 4, 2114-2133
- **K. HARADA et al.** *International Journal of Hydrogen Energy,* 2020, vol. 45, 31389-31 395
- **H. SATO et al.** *International Journal of Hydrogen Energy,* 2021, vol. 46, 33 689-33 695
- **KRISTIAN STANGELAND ; HAILONG LI ; ZHIXIN YU.** *Energy, Ecology and Environment,* 2020, vol. 5, 272-285
- **N.KANOUN et al.** Catalytic properties of Cu based catalysts containing Zr and/or V for methanol synthesis from a carbon dioxide and hydrogen mixture. *CATALYSIS LETTERS,* 1992, vol. 15, 231-235
- **R. M. NAVARRO et al.** Methanol Synthesis from CO2: A Review of the Latest Developments in Heterogeneous Catalysis. *Materials,* 2019, vol. 12, 3902
- Catalytic carbon dioxide hydrogenation to methanol: A review of recent studies. *chemical engineering research and design,* 2014, vol. 92, 2557-2567
- *Angew. Chem. Int. Ed.,* 2016, vol. 55, 6261-6265
- Acetaldehyd. **MARC ECKERT ; GERALD FLEISCHMANN ; REINHARD JIRA ; HERMANN M. BOLT ; KLAUS GOLKA.** Ullmann's Encyclopedia of Industrial Chemistry. vol. 1, 197
- Vinyl Esters. **G. ROSCHER.** Ullmann's Encyclopedia of Chemical Technology. John Wiley & Sons, 2007
- Esterification. **ASLAM, M. ; TORRENCE, G.P ; ZEY, E.G.** Kirk-Othmer Encyclopedia of Chemical Technology. 2000, vol. 10, 471
- Acetic Acid. **LE BERRE, C. ; SERP, P. ; KALCK, P. ; TORRENCE, G.P.** Ullmann's Encyclopedia of Industrial Chemistry. 2014, 25
- Acetic Anhydride and Mixed Fatty Acid Anhydrides. **HELD, H. ; RENGSTL, A. ; MAYER, D.** Ullmann's Encyclopedia of Industrial Chemistry. 2000, vol. 1, 248
- Acetic Anhydride and Mixed Fatty Acid Anhydrides. **HELD, H. ; RENGSTL, A. ; MAYER, D.** Ullmann's Encyclopedia of Industrial Chemistry. 2000, vol. 1, 245
- Acetic Anhydride and Mixed Fatty Acid Anhydrides. **HELD, H. ; RENGSTL, A. ; MAYER, D.** Ullmann's Encyclopedia of Industrial Chemistry. 2000, vol. 1, 244
- Poly(vinyl esters). **RINNO, H.** Ullmann's Encyclopedia of Industrial Chemistry. 2000, vol. 28, 477
- **DOMINIC BYRNE et al.** Biodegradability of polyvinyl alcohol based film used for liquid detergent capsules. *Tenside Surf. Det.,* 2021, vol. 58, 2
- **E. CHIELLINI et al.** *Prog. Polym. Sci.,* 2003, vol. 28, 963-1014